# EUROPEAN PATENT APPLICATION

(11) **EP 4 717 295 A1**
(43) Date of publication of application: **01.04.2026**
(21) Application number: 24882606.7
(22) Date of filing: 13.08.2024
(51) Int. Cl.: A61M 21/02, A61H 23/02, A61H 15/00, A61B 5/00, G16H 20/70, A61M 21/00

(54) **SMART SLEEP INDUCTION SYSTEM**

(30) Priority: 26.10.2023 KR 20230144848
(71) Applicant: Glonity Inc., Incheon 21990 (KR)
(72) Inventor: SEO, Seungwoo, Incheon 21911 (KR)
(74) Representative: Lichtnecker, Markus Christoph
(86) International application number: PCT/KR2024/012038
(87) International publication number: WO 2025/089577

(57) **Abstract**

A smart sleep induction system is disclosed. The system includes a smart massage device including a massage unit pressing the body of a user, and a user app playing ASMR sound sources for sleep induction by being executed in a mobile terminal, in which the smart massage device further includes a sound source receiver receiving ASMR sound sources played by the mobile terminal, a sound source analyzer analyzing frequencies of received source sources, and a massage controller differently controlling pressure that is applied to a body by controlling the massage unit in accordance with analyzed frequencies.

## Description

### TECHNICAL FIELD

The present disclosure relates to a system for inducing sleep of a user.

### BACKGROUND ART

A sound source providing system for improving sleep quality on the basis of artificial intelligence has been disclosed in Korean Patent No. 10-2260010. This system classifies the risk of sleep disorder of examinees into four groups through unsupervised machine learning by inputting sleep-related data of users through a survey method that is standardized for improvement of sleep of users, whereby the system provides users with combinations of ASMR sound sources, poetry recitation sound sources, and sound sources composed using an AI algorithm and measures the brain waves of users so that sleep disorders of users can be removed in real time.

### DETAILED DESCRIPTION OF INVENTION

### TECHNICAL PROBLEMS

An objective of the present disclosure is to provide a technical solution that can induce sleep of a user and help the user to have a deep sleep by combining not only ASMR sound sources, but a massage function.

### TECHNICAL SOLUTION

A smart sleep induction system according to an aspect may include a smart massage device including a massage unit pressing the body of a user, and a user app playing ASMR sound sources for sleep induction by being executed in a mobile terminal. Further, the smart massage device may further include a sound source receiver receiving ASMR sound sources played by the mobile terminal, a sound source analyzer analyzing frequencies of received source sources, and a massage controller differently controlling pressure that is applied to a body by controlling the massage unit in accordance with analyzed frequencies.

The massage unit may include a vibrator massage unit including one or more vibrators for vibration massage. Further, when a sound source frequency corresponds to a high frequency band, the massage controller may increase a vibration frequency and decrease vibration intensity in comparison to when the sound source frequency corresponds to a low frequency band, and when a sound source frequency corresponds to a low frequency band, the massage controller may decrease a vibration frequency and increase vibration intensity in comparison to when the sound source frequency corresponds to a high frequency band.

The smart sleep induction system may further include a wearable device for measuring a sleep state of a user. The user App, through the wearable device, may decrease volume of a playback sound source from a default volume level to a first volume level when it is determined that an awake state of a user has changed into a REM (rapid eye movement) sleep state, decrease the volume of the playback sound source to a second volume level when it is determined that the REM sleep state has changed into a non-REM (non-rapid eye movement) sleep state, and stop playing the sound source when it is determined that a slow wave sleep state has been entered in the non-REM sleep state.

The smart massage device may be configured as a pillow including an occiput support section (10) where the occipital region of a user rests and a cervical vertebra support section (30) extending downward from the occiput support section (10) and supporting the vertebrae of a user, and the massage unit (210) may include a vibrator massage unit (211) disposed inside the occiput support section (10) and configured as a vibration pad (40) having a two-dimensional array of a plurality of vibrators, and an acupressure ball massage unit (212) disposed inside the cervical vertebra support section (30) and including a plurality of acupressure balls (51).

The acupressure ball massage unit (212) may include a first acupressure part (212a) and a second acupressure part (212b) disposed at both sides of the vertebrae of a user lying in a supine position, and the first acupressure part (212a) and the second acupressure part (212b) each may include an acupressure module (50) formed in a shape similar to a frustoconical shape, having a plurality of acupressure balls (51) circumferentially on a side, and rotating about a center axis defining a height direction.

### EFFECT OF INVENTION

The present disclosure creates an effect that enables improved sleep and deep sleep induction for a user by integrating ASMR sound sources with physical massage.

### BRIEF DESCRIPTION OF THE DRAWING

FIG. 1 is a block diagram of a smart sleep induction system according to an embodiment.
FIG. 2 is a perspective view showing a smart massage device according to an embodiment.
FIG. 3 is a plan view showing the smart massage device according to an embodiment.
FIG. 4 is an exemplary view showing the state when the head and neck of a user rest on the smart massage device according to an embodiment.
FIG. 5 is a plan view showing the structure of an acupressure ball massage unit applied to the smart massage device according to an embodiment.
FIG. 6 is a plan view showing the structure of the acupressure ball massage unit applied to the smart massage device according to an embodiment.
FIG. 7 is an exemplary view showing expansion of the acupressure ball massage unit applied to the smart massage device according to an embodiment.
FIG. 8 is an exemplary view showing that earphones and a mobile device can be connected to the smart massage device according to an embodiment.

### BEST MODE FOR CARRYING OUT THE INVENTION

Aspects of the present disclosure that were described above and will be added below will be made clearer through exemplary embodiments that are described with reference to the accompanying drawings. Hereafter, such embodiments of the present disclosure are described in detail so that those skilled in the art can easily understand and achieve the present disclosure.

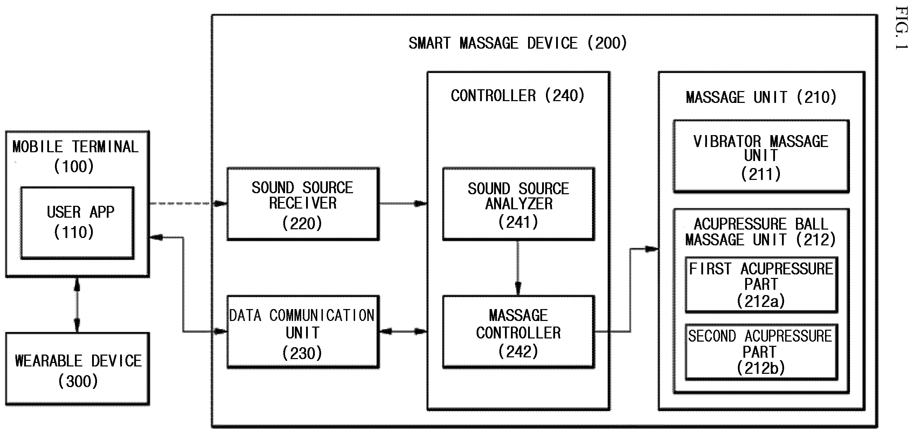

FIG. 1 is a block diagram of a smart sleep induction system according to an embodiment and the smart sleep induction system includes at least some of the components shown in FIG. 1. A mobile terminal 100 is a computing device that can perform mobile communication, and for example, may be a smartphone. A user app 110 is installed and executed in the mobile terminal 100 and the user app 110 plays and outputs ASMR (autonomous sensory meridian response) sound sources for inducing sleep of users. ASMR sound sources may mean all sound sources that can be used for various purposes, such as mental stability, stress relief, sleep, and meditation, on the basis of sounds, and particularly, may mean only sound sources that are used for sleep.

A smart massage device 200 is for helping induction of sleep and providing deep sleep by stretching muscles by stimulating the body of users in accordance with ASMR sound sources that are played. A wearable device 300, which is supposed to be worn on the body of a user, may be a well-known smart watch. The wearable device 300 may include an acceleration sensor, a 3-axial gyroscope sensor, and an optical heart rate sensor, and the sleep state of a user can be measured using signals sensed by these sensors. As well known, a sleep state is classified into REM (rapid eye movement) sleep and non-RRM (non-rapid eye movement) sleep, and non-REM sleep is classified into first to fourth stages. Non-REM sleep includes a stage called slow wave sleep, which is third to fourth stages. Since it has been well known that the wearable device 300 measures whether a user is in an awake state or a sleep state, whether a user is in a REM sleep state or a non-REM sleep state when the user is in a sleep state, and whether a user has entered slow wave sleep when the user is in a non-REM sleep state, it is not described in detail. Further, the wearable device 300 can measure the stress index of a user, which has also been well known similar to sleep measurement.

The smart massage device 200 is described again. As shown in FIG. 1, the smart massage device 200 includes a massage unit 210, a sound source receiver 220, and a controller 240, and may further include a data communication unit 230. The massage unit 210, which is for stretching muscles by pressing the body of a user, includes a vibrator massage unit 211 that presses body parts of a user by vibrating one or more vibrators, and may include an acupressure ball massage unit 212 that presses body parts of a user using one or more acupressure balls. In an embodiment, the vibrator massage unit 211 is for massaging the occipital region of a user and the acupressure ball massage unit 212 is for massaging the upper trapezius muscles of a user. These massage units may be integrated and may be designed in the form of a pillow to facilitate sleeping without interfering with sleep and configured to be able to massage a wide region including shoulders and a neck. This will be described below.

The sound source receiver 220 receives ASMR sound sources that are played by the user app 110 of the mobile terminal 100. In an embodiment, the sound source receiver 220 is a component that receives sounds, which are output through a speaker of the mobile terminal 100, through a microphone. In another embodiment, the sound source receiver 220 is a wired interface connected to the mobile terminal 100 through a wire and receiving playback sound sources from the mobile terminal 100. In this case, the smart massage device 200 can output playback sound sources, which are received through a wired interface, to a user through an audio output device (a speaker, earphones, or the like) thereof. Further, the data communication unit 230 is a component for transmitting/receiving data through short-range wireless communication with the mobile terminal 100, and for example, may be a Bluetooth module, etc.

The controller 240, which is a controller that generally controls the smart massage device 200, may include a sound source analyzer 241 and a massage controller 242. The sound source analyzer 241 analyzes the frequency of received sound sources. The sound source frequency analysis technique has been well known in the art, so it is not described in detail. The massage controller 242 controls the massage unit 210 such that the body of a user is pressed, and controls pressure such that different pressures are applied to a body in accordance with the frequencies analyzed by the sound source analyzer 241. For example, the intensity of pressing is differently controlled or the vibration cycle is differently controlled. Further, the smart massage device 200 can simultaneously control the vibrator massage unit 211 and the vibration ball massage unit 210.

Hereafter, the massage controller 242 targeting the vibrator massage unit 211 is described in detail. In an embodiment, when a sound source frequency analyzed through the sound source analyzer 241 corresponds to a predetermined high frequency band, the massage controller 242 can increase a vibration frequency and decrease vibration intensity in comparison to when the sound source frequency corresponds to a low frequency band, and when a sound source frequency corresponds to a predetermined low frequency band, the massage controller 242 can decrease the vibration frequency and increase the vibration intensity in comparison to when the sound source frequency corresponds to a high frequency band. This is for enabling a user to balance the body rhythm to help induction of sleep by synchronizing aural stimulation and physical stimulation to some extent. Further, this prevents stimulation from being excessively applied by reducing the vibration intensity while the number of times of vibration is high.

Meanwhile, the user app 100 can differently control the volume level of ASMR sound sources in accordance with the sleep state of a user. In an embodiment, the user app 110 can set the volume level for playing an ASMR sound source as a default volume level when a user is in an awake state, and can decrease the default volume level when the awake state changes into a sleep state. That is, this is for preventing the magnitude of volume from interfering with sleep and for helping maintain sleep of the user with appropriate volume when a user enters sleep.

In a detailed embodiment, the user app 110 decreases the volume of a playback sound source from a default volume level to a first volume level when it is determined that the state of a user has changed into a REM sleep state from an awake state, and decreases the volume of the playback sound source from the first volume level to a second volume level when it is determined that the REM sleep state has changed into a non-REM sleep state. Further, when it is determined that a slow wave sleep state has been entered after the non-REM sleep state, the user app stops playing the ASMR sound source. That is, the volume of a sound source is decreased step by step in accordance with the sleep state of a user, and when the user enters deep sleep, playback of the sound source is stopped. Accordingly, it is possible to expect induction of more effective sleep and deep sleep.

In an additional embodiment, the sound source analyzer 241 measures the decibel of sound sources received through the sound source receiver 220. Further, when it is determined that a measured decibel is a numerical value corresponding to a first volume level, the massage controller 242 determines that the state of the user has changed into a REM sleep state from an awake state, and decreases the vibration intensity of the vibrator massage unit 211. Next, when it is determined that a measured decibel is a numerical value corresponding to a second volume level, the massage controller 242 determines that the state of the user has changed into a non-REM sleep state from the REM sleep state, and additionally decreases the vibration intensity of the vibrator massage unit 211. Finally, when a sound source is no longer received through the sound source receiver 220, the massage controller 242 determines that the user has entered a slow wave sleep state, and inactivates the vibrator massage unit 211. That is, operation of a vibrator is stopped. According to the above description, it becomes possible to reduce stimulation step by step in accordance with the depth of sleep of a user, so it becomes possible to help sleep and deep sleep of a user.

The vibrator massage unit 211 according to an embodiment may be configured by two-dimensionally arranging a plurality of vibrators to apply vibration massage to the occipital region of a user. Further, the massage controller 242 can increase or decrease the massage area for an occipital region by controlling operation of some or all of the vibrators of the vibrator massage unit 211. For example, the massage controller 242 maximizes the massage area for an occipital region by activating all of the vibrators of the vibrator massage unit 211 when a user is in an awake state, and decreases the massage area for the occipital region by inactivating some of the vibrators when the awake state changes into a REM sleep state. Further, when the REM sleep state changes into a non-REM sleep state, the massage controller additionally decreases the massage area for the occipital region by inactivating some of the vibrators that are in an activated state, and the massage controller stops massaging the occipital region by inactivating all of the activated vibrators when it is determined that a slow wave sleep has been entered.

Meanwhile, when an ASMR sound source is played, the user app 110 can transmit the frequency response information of the ASMR sound source to the smart massage device 200 using a short-range wireless communication technology. To this end, the user app 110 can analyze the frequency response of an ASMR sound source in advance or when the ASMR sound source is played. The smart massage device 200 receives the frequency response information of ASMR sound sources and the received frequency response information can be used in the sound source analyzer 241 to extract only playback sound sources of the mobile terminal 100 from sounds received through the sound source receiver 220. This is for effectively separating noises when sounds (noises) other than ASMR sound sources are received.

Next, the massage controller 242 targeting the acupressure ball massage unit 222 is described in detail. First, the acupressure ball massage unit 222 may include a first acupressure part 212a and a second acupressure part 212b, and the first acupressure part 212a and the second acupressure part 212b may be configured to come in contact with the upper left trapezius muscle and the upper right trapezius muscle from the cervical vertebrae, respectively, when a user is lying down. The first acupressure part 212a is composed of a plurality of acupressure balls arranged in a circular shape and arrange in order of large size (increasing) in a first direction (e.g., counterclockwise), and, on the contrary, the second acupressure part 212b is composed of a plurality of acupressure balls arranged in order of small size (decreasing) in a second direction (e.g., clockwise), and the first acupressure part 212a and the second acupressure part 212b are configured to rotate in opposite directions by operation of a common motor.

In an embodiment, when a sound source frequency analyzed through the sound source analyzer 241 corresponds to a high frequency band, the massage controller 242 rotates the first acupressure part 212a in the first direction and rotates the second acupressure part 212b in the second direction by operating the motor such that the acupressure intensity is increased in order from the smallest acupressure ball to the largest acupressure ball for both of the upper left trapezius muscle part and the upper right trapezius muscle part. In contrast, when a sound source frequency analyzed through the sound source analyzer 241 corresponds to a predetermined low frequency band, the massage controller 242 rotates the first acupressure part 212a in the second direction and rotates the second acupressure part 212b in the first direction by operating the motor such that the acupressure intensity is decreased in order from the largest acupressure ball to the smallest acupressure ball for both of the upper left trapezius muscle part and the upper right trapezius muscle part.

In an additional embodiment, the massage controller 242 stops operating the first acupressure part 212a and the second acupressure part 212b when the state of a user changes from an awake state to a sleep state. This is for preventing interference with sleep of a user by stopping movement of the upper body due to the acupressure balls.

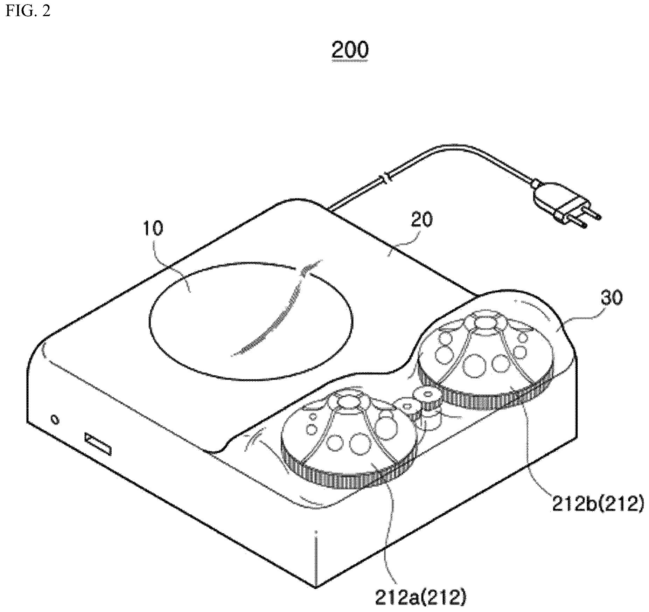

Meanwhile, the smart massage device 200, as shown in FIG. 2 to FIG. 4, may be configured as a pillow, and for example, may include an occiput support section 10, a temporal support section 20, and a cervical vertebra support section 30.

The occiput support section 10, which is the part where the user's occipital region rests in a supine position, may be formed flat, but it is preferable that the occiput support section 10 is concavely recessed so that the user's occipital region stably rests therein. The temporal support section 20, which is the part where a temporal region of a user lying sideways rests, extends from both sides of the occiput support section 10 and may be formed flat.

The cervical vertebra support section 30, which is the part that supports the cervical vertebrae of a user lying in a supine position or sideways and with which the trapezius muscles of the user are in contact, extends downward from the occiput support section 10 and the temporal support section 20. Assuming that the direction that a user lying in a supine position faces is the forward direction, the cervical vertebra support section 30 is formed convexly forward further than the occiput support section 10 and the temporal support section 20, thereby being able to induce a forward curve of the cervical vertebrae of a user lying in a supine position.

The smart massage device 200 includes the massage unit 210 that presses the body of a user, and the massage unit 210 may include at least one of the vibrator massage unit 211 that presses body parts of a user by vibrating one or more vibrators and the acupressure ball massage unit 212 that presses body parts of a user using one or more acupressure balls.

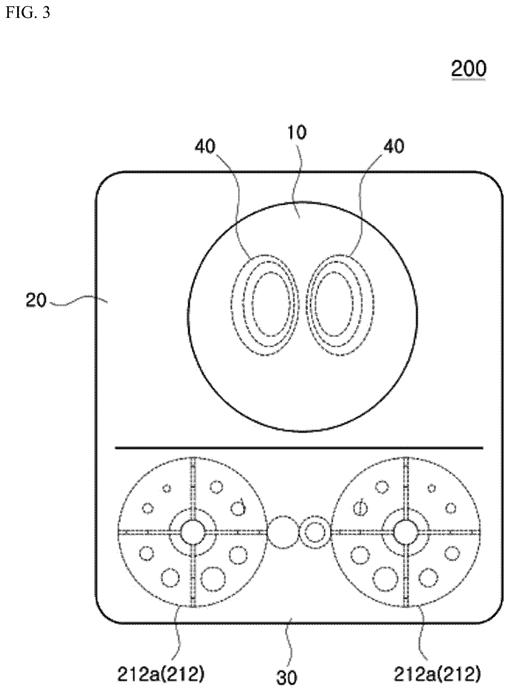

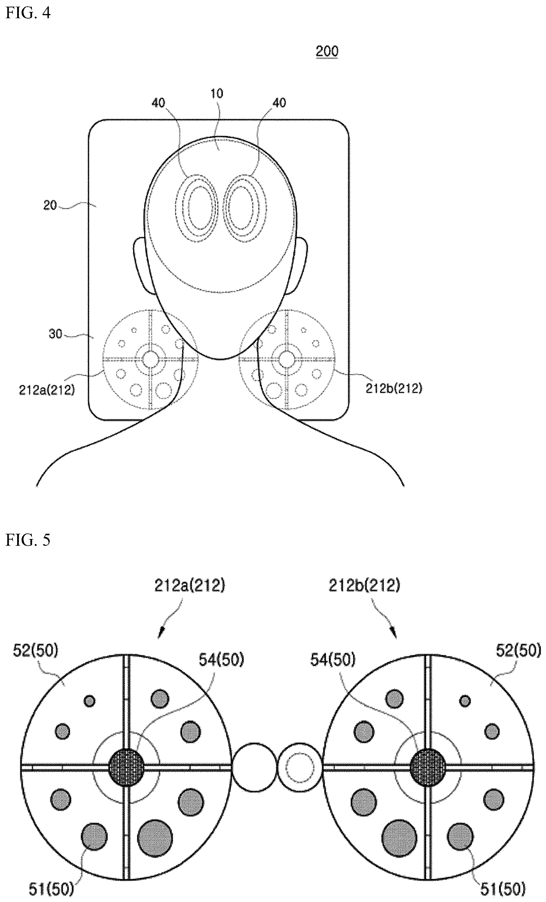

For example, as shown in FIG. 3 and FIG. 4, the vibrator massage unit 211 may be provided on the occiput support section 10 and the vibrator massage unit 211 may be a vibration pad 40 disposed inside the occiput support section 10 and having a two-dimensional array of a plurality of vibrators. Not only the vibration frequency and the vibration intensity, but the vibration range of the vibration pad 40 can be adjusted in accordance with the sound source frequencies analyzed by the massage controller 242. Further, a plurality of vibration pads 40 may be disposed inside the occiput support section 10, and for example, two vibration pads 40 may be symmetrically disposed at the left and right sides.

As another example, as shown in FIG. 2 to FIG. 4, the acupressure ball massage unit 212 may be provided inside the cervical vertebra support section 30. The acupressure ball massage unit 212 includes a first acupressure part 212a and a second acupressure part 212b disposed at both sides of the vertebrae of a user lying in a supine position, and the first acupressure part 212a and the second acupressure part 212b, as shown in FIG. 5, each may include a plurality of acupressure balls that can apply acupressure to the trapezius muscles of a user.

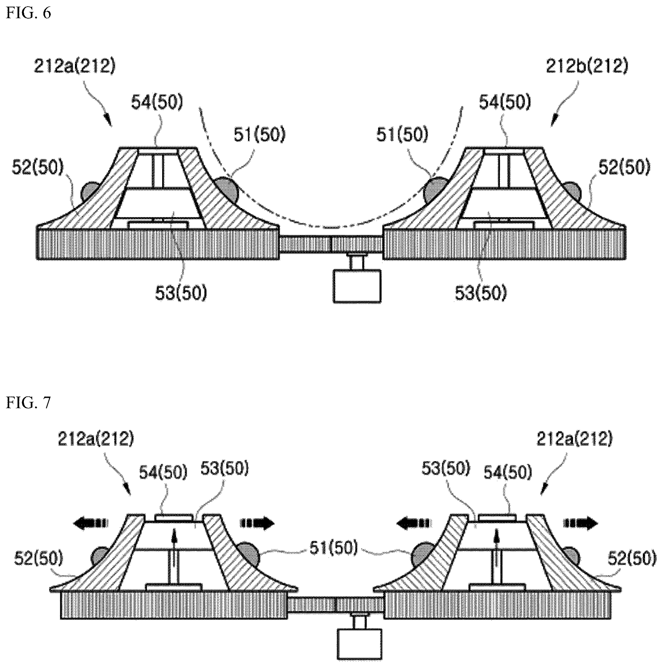

As a detailed example, the first acupressure part 212a and the second acupressure part 212b, as shown in FIG. 6, may include an acupressure module 50 formed in a shape that is the same as/similar to a frustoconical shape and having a plurality of acupressure balls 51 circumferentially disposed on the side. The acupressure module 50 can rotate about a center axis defining a height direction, and the acupressure modules 50 of the first acupressure part 212a and the second acupressure part 212b may be configured to be rotated by power from individual power sources and may be configured to be rotated by power from one power source. Further, the acupressure modules 50 of the first acupressure part 212a and the second acupressure part 212b can be rotated in the same direction, but are preferably configured to be rotated in opposite directions to increase the acupressure effect by the acupressure balls 51. For example, the acupressure module 50 of the first acupressure part 212a may be rotated clockwise and the acupressure module 50 of the second acupressure part 212b may be rotated counterclockwise, thereby stimulating trapezius muscles downward, or the acupressure module 50 of the first acupressure part 212a may be rotated counterclockwise and the acupressure module 50 of the second acupressure part 212b may be rotated clockwise, thereby stimulating trapezius muscles upward.

It is preferable that the acupressure modules 50 configured to rotate in this way, as shown in FIG. 6, are formed such that the side forms a concave curved surface so that the neck part of a user is stably and comfortably supported by the side of the acupressure module 50 of the first acupressure part 212a and the side of the acupressure module 50 of the second acupressure part 212b. The plurality of acupressure balls 51 disposed on the side of the acupressure module 50 may be disposed with predetermined gaps therebetween while convexly protruding from the side of the acupressure module 50, may be formed in the same size, and may be formed in different sizes as well. For example, as shown in FIG. 5, the plurality of acupressure balls 51 disposed on the side of the acupressure module 50 of the first acupressure part 212a may be arranged such that the sizes increase in one direction (counterclockwise) and the plurality of acupressure balls 51 disposed on the side of the acupressure module 50 of the second acupressure part 212b may be arranged such that the sizes increase in another direction (clockwise).

Further, the side of the acupressure module 50 on which the plurality of acupressure balls 51 is disposed, as shown in FIG. 5, may be radially divided and composed of a plurality of supporting segments 52, and an acupressure force adjuster 53 that laterally moves the plurality of supporting segments 52 by moving up and down may be disposed inside the acupressure module 50, as shown in FIG. 6. For example, the acupressure force adjuster 53 is formed on a frustoconical shape and can move up and down in the acupressure module 50, and the inner surfaces of the plurality of supporting segments 52 may be formed to make curved surfaces corresponding to the acupressure force adjuster 53 that is in contact with the inner surfaces. Further, the plurality of supporting segments 52 may be connected to each other to simultaneously move outward or inward and may be coupled to the bottom of the acupressure module 50 to be able to move outward and inward.

Accordingly, as shown in FIG. 7, when the acupressure force adjuster 53 is moved upward, the plurality of supporting segments 52 is moved outward, so the size of the acupressure module 50 is increased, whereby the acupressure force by the acupressure balls 51 can be increased. Further, when the acupressure force adjuster 53 is moved downward, the plurality of supporting segments 52 is moved inward, so the size of the acupressure module 50 is decreased, whereby the acupressure force by the acupressure balls 51 can be decreased.

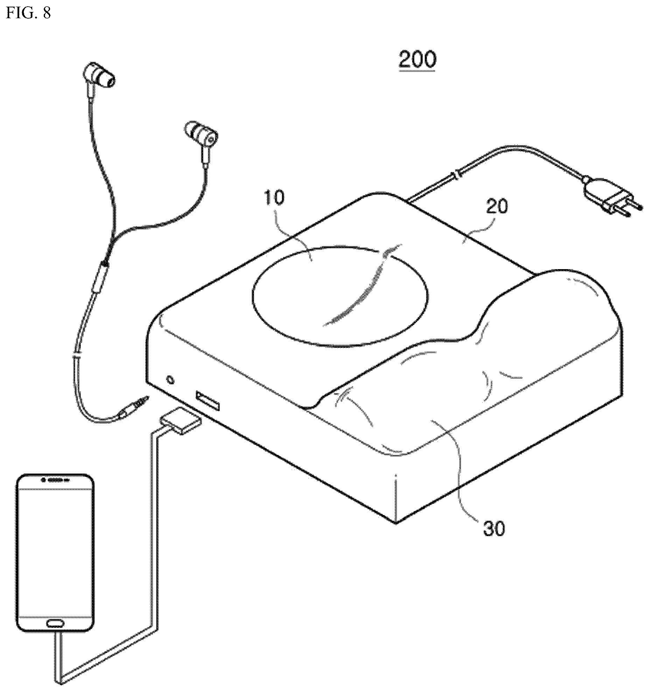

A speaker 54 may be disposed on the upper end of the acupressure module 50 described above, as shown in FIG. 5 and FIG. 6, and ASMR sound sources for inducing sleep of a user can be output through the speaker 54. In order that output can be made through earphones in addition to the speakers 54, the smart massage device 200, as shown in FIG. 8, may have a sound output terminal to which earphones can be connected in a wired type or may have a Bluetooth transceiver to which earphones can be connected in a wireless type. Further, the smart massage device 200, as shown in FIG. 8, may also have a power terminal that can supply power to the mobile terminal 100 of a user, etc.

Preferred embodiments of the present disclosure were described. It would be understood by those skilled in the art that the present disclosure may be modified with departing from the scope of the present disclosure. Therefore, the disclosed embodiments should be considered in terms of explaining, not limiting. The scope of the present disclosure is not shown in the above description, but claims, and all differences within an equivalent range should be construed as being included in the present disclosure.

## Claims

1. A smart sleep induction system comprising:
a smart massage device comprising a massage unit pressing the body of a user; and a user app playing ASMR sound sources for sleep induction by being executed in a mobile terminal,
wherein the smart massage device further comprises:
a sound source receiver receiving ASMR sound sources played by the mobile terminal;
a sound source analyzer analyzing frequencies of received source sources; and
a massage controller differently controlling pressure that is applied to a body by controlling the massage unit in accordance with analyzed frequencies.

2. The smart sleep induction system of claim 1, wherein the massage unit comprises a vibrator massage unit comprising one or more vibrators for vibration massage, and
when a sound source frequency corresponds to a high frequency band, the massage controller increases a vibration frequency and decreases vibration intensity in comparison to when the sound source frequency corresponds to a low frequency band, and when a sound source frequency corresponds to a low frequency band, the massage controller decreases a vibration frequency and increases vibration intensity in comparison to when the sound source frequency corresponds to a high frequency band.

3. The smart sleep induction system of claim 2, further comprising a wearable device for measuring a sleep state of a user,
wherein the user app, through the wearable device, decreases volume of a playback sound source from a default volume level to a first volume level when it is determined that an awake state of a user has changed into a REM (rapid eye movement) sleep state, decreases the volume of the playback sound source to a second volume level when it is determined that the REM sleep state has changed into a non-REM (non-rapid eye movement) sleep state, and stops playing the sound source when it is determined that a slow wave sleep state has been entered in the non-REM sleep state.

4. The smart sleep induction system of claim 3, wherein the sound source analyzer measures the decibel of playback sound sources, and
the massage controller decreases the vibration intensity when it is determined that a measured decibel is a numerical value corresponding to the first volume level, additionally decreases the vibration intensity when a measured decibel is a numerical value corresponding to the second volume level, and stops operation of the vibrators when the sound source receiver finishes receiving sound sources.

5. The smart sleep induction system of any one of claims 2 to 4, wherein the vibrator massage unit comprises a plurality of vibrators two-dimensionally arranged to apply vibration massage to the occipital region of a user, and
the massage controller maximizes a massage area for an occipital region by activating all of the vibrators of the vibrator massage unit when a user is in an awake state, controls and inactivates some of the vibrators such that the massage area for the occipital region is decreased when it is determined that the awake state has changed into the REM sleep state, additionally controls and inactivates some of the vibrators such that the massage area for the occipital region is additionally decreased when it is determined that the REM sleep state has changed into the non-REM sleep state, and controls and inactivates all of activated vibrators to stop massaging the occipital region when it is determined that the slow wave sleep state has been entered.

6. The smart sleep induction system of claim 5, wherein the massage unit further comprises an acupressure ball massage unit that comprises a first acupressure part comprising a plurality of acupressure balls arranged in a circular shape to come in contact with an upper left trapezius muscle and arranged such that sizes increase in a first direction, and a second acupressure part comprising a plurality of acupressure balls arranged in a circular shape to come in contact with an upper right trapezius muscle and arranged such that sizes increase in a second direction; and
when a sound source frequency corresponds to a high frequency, the massage controller rotates the first acupressure part in the first direction and rotates the second acupressure part in the second direction such that acupressure intensity is increased in order from a smallest acupressure ball to a largest acupressure ball for both of an upper left trapezius muscle part and an upper right trapezius muscle part, and when a sound source frequency corresponds to a low frequency, the massage controller rotates the first acupressure part in the second direction and rotates the second acupressure part in the first direction such that acupressure intensity is decreased in order from the largest acupressure ball to the smallest acupressure ball for both of the upper left trapezius muscle part and the upper right trapezius muscle part.

7. The smart sleep induction system of claim 1, wherein the smart massage device is configured as a pillow comprising an occiput support section (10) where the occipital region of a user rests and a cervical vertebra support section (30) extending downward from the occiput support section (10) and supporting the vertebrae of a user, and
the massage unit (210) comprises a vibrator massage unit (211) disposed inside the occiput support section (10) and configured as a vibration pad (40) having a two-dimensional array of a plurality of vibrators, and an acupressure ball massage unit (212) disposed inside the cervical vertebra support section (30) and comprising a plurality of acupressure balls (51).

8. The smart sleep induction system of claim 7, wherein the acupressure ball massage unit (212) comprises a first acupressure part (212a) and a second acupressure part (212b) disposed at both sides of the vertebrae of a user lying in a supine position, and
the first acupressure part (212a) and the second acupressure part (212b) each comprise an acupressure module (50) formed in a shape similar to a frustoconical shape, having a plurality of acupressure balls (51) circumferentially on a side, and rotating about a center axis defining a height direction.

9. The smart sleep induction system of claim 8, wherein the acupressure module (50) of the first acupressure part (212a) and the acupressure module (50) of the second acupressure part (212b) are rotated in opposite directions.

10. The smart sleep induction system of claim 8, wherein the plurality of acupressure balls (51) disposed on the side of the acupressure module (50) is formed in different sizes, and
the plurality of acupressure balls (51) disposed on the side of the acupressure module (50) of the first acupressure part (212a) is arranged such that sizes increase in a first direction, and the plurality of acupressure balls (51) disposed on the side of the acupressure module (50) of the second acupressure part (212b) is arranged such that sizes increase in a second direction.

11. The smart sleep induction system of claim 8, wherein the acupressure module (50) is radially divided on the side and is composed of a plurality of supporting segments (52), has an acupressure force adjuster (53) therein that laterally moves the plurality of supporting segments (52) by moving up and down, so when the acupressure force adjuster (53) is moved upward, the plurality of supporting segments (52) is moved outward, whereby a size of the acupressure module (50) is increased, and when the acupressure force adjuster (53) is moved downward, the plurality of supporting segments (52) is moved inward, whereby the size of the acupressure module (50) is decreased.
